# EUROPEAN PATENT SPECIFICATION

(11) **EP 0 327 633 B1**
(45) Date of publication and mention of the grant of the patent: **18.11.1993**
(21) Application number: 88907355.7
(22) Date of filing: 11.08.1988
(51) Int. Cl.: G01N 33/58, G01N 33/532, G01N 33/533, G01N 33/577, C12N 13/00, C12N 5/00, C12P 21/00

(54) **PROCEDURE FOR RELIEVING CELL MIXTURES AND TISSUES OF UNWANTED POPULATIONS**
VERFAHREN ZUR BEFREIUNG VON ZELLGEMISCHEN UND GEWEBEN VON UNERWÜNSCHTEN POPULATIONEN
PROCEDE PERMETTANT DE SOULAGER DES MELANGES CELLULAIRES ET DES TISSUS DE POPULATIONS INDESIRABLES

(30) Priority: 12.08.1987 HU 365187
(43) Date of publication of application: 16.08.1989
(73) Proprietor: NEMETH, Péter, H-7636 Pécs (HU); BERKI, Timea, H-7636 Pécs (HU)
(72) Inventor: NEMETH, Péter, H-7636 Pécs (HU); BERKI, Timea, H-7636 Pécs (HU)
(74) Representative: Schwabe - Sandmair - Marx
(86) International application number: HU8800057
(87) International publication number: WO8901630

(56) References cited:
- EP-A- 0 218 352
- WO-A-86/02734
- GB-A- 2 063 469
- HUNNIUS PHARMAZEUTISCHES WÖRTERBUCH, 1986, Buchstabe H; Seite 1*
- LEHRBUCH DER ORGANISCHEN CHEMIE, Prof.Dr. Hans Beyer, 18. Aufl., 1978; Seiten 622-624*

## Description

### Technical field

The present invention relates to a conjugate comprising a monoclonal antibody and a tag, to a method for preparing the conjugate and to the use of the conjugate in a method for destroying unwanted cells in a cell population.

### Background art

The targeted and selective elimination of certain cell types from mixed cell populations or from tissues causes problems both in the experimental research work and in the clinical practice. For this purpose the application of carrier-bound, i. e. immunoglobuline-bound cytotoxic substances was attempted; see for example Keith, A. et al. Cancer Imm. Immunother. (1981) 12, 39 - 41. This constitutes the theoretical background of the so called targeting therapy in the course of which monoclonal antibodies are conjugated with strongly cytotoxic substances, like alpha chain of ricin. The selectivity of the method depends solely on the specificity of the carrier molecule. Since it has been proven for more and more antibodies which have been earlier regarded as specific for a single cell type and since it was found that the antigen determinant bound by the antibodies may be present also on other structures, specificity is considered to be rather quantitative than qualitative. To eliminate the effect of unwanted binding the combination of locally effective physical effects may offer a solution. This underlines the importance of normally harmless molecules which become toxic only by physical influence, e. g. by light irradiation.

Phototherapy is a field of growing importance in medicine. Among the applications of light sources directed towards specific targets, procedures using laser beams have received special attention. Beside the large power density, the unique physical properties (coherency, polarisation) of the laser beam add greatly to the elicited biological effect.

In the course of testing the biological effects of a low power He-Ne laser on in vitro cell cultures we could demonstrate a definite energy dependent response. At a given irradiation energy level biological activation takes place while over a higher threshold level cell disruption can be observed. This phenomenon is described in the Kisérketes Orvostudomány (1984) 36, 96 and the Studia Biophys. (1985) 105, 144. This method is called immunotargeting.

By applying different photosensibilising molecules the energy threshold values of biological activation and cell disruption can be influenced.

It is known from the prior art that phorphyrin derivatives can be well activated at the wavelength range of visible light and therefore they are extensively used as photosensibilisers (see for example Br. J. Cancer (1979) 39, 398; Photochem. Photobiophys. (1988) 10, 53 - 59 and Cancer Res. (1981) 41, 401.)

GB-A 2,063,469 discloses a conjugate for use in the detection and quantification of antibodies and antigens in body fluids which comprises an immunologically active compound having attached to it a tag in the form of a metallo porphyrin which metallo porphyrin is capable of catalyzing a chemiluminescent reaction. Apparently, the compounds used as the tag contain coordinated metal atoms.

### Disclosure of the Invention

The present invention is based on the observation that by contacting the surface of various cells with photosensibilising substances including the by itself non toxic and by our He-Ne laser directly non-activable hematoporphyrin derivatives (HPD-molecules), followed by irradation with laser beam, results in cell disruption already at an energy level where non-sensibilised cells would not be destroyed.

To attain this goal prophyrin derivatives, preferably hematoporphyrin hydrochloride is covalently conjugated to monoclonal antibodies capable of selectively binding to structures on the cell surface.

Conjugation is performed by incubation of the monoclonal antibody protein with the porphyrin derivative, preferably with hematoporphyrin hydrochloride In an aqueous solution.

Irradiation of these hematoporphyrin derivatives by visible ligth results in a wave length dependent free radical induction process that causes damage to the biological structures. Since the absorption maximum of hematoporphyrin is around 405 nm a large amount of free radicals is produced and their damaging effect on living cells cannot be controlled after irradiation at this wavelength. In contrast to this the effect is greatly reduced at the wavelength range around 630 nm since the energy of light is absorbed by the biological structure itself rather than by the hematoporphyrin molecule and by virtue of electron transfer processes a much more limited effect is brought about, amenable to more efficient regulation. The role of photosensibiliser molecules in this process is to bring forth electron transfer.

The application of He-Ne laser with 632.8 nm wavelength of emitted light allows for the relatively short term concentrated photoenergy flux sufficient for initiating regulated electron transfer processes but not leading directly to free radical generation.

In one aspect of the present invention there is provided a process for the production of photosensibilising conjugates comprising the incubation of for example anti-PNAr-I, anti-PNar-II, anti-PNAr-III, anti-WGA, anti-T3, anti-AFP, anti-HCG and anti-H antibodies with certain porphyrine derivatives in an aqueous medium.

The characteristics of the above mentioned target-specific monoclonal antibodies are as follows:

### anti-PNAr-I

A monoclonal antibody (IgG1) which reacts with pea-nut agglutinin antigen (PNA) binding receptor isolated from the ephithelial cells of the gastric mucosa by lectin affinity chromatography. It reacts with the Golgi region of the cells in the normal gastric mucosa as well as with surface and cytoplasmic structures on gastric cancerous cells. The latters specifically bind the monoclonal antibody also in their metastases.

### anti-PNAr-II

A monoclonal antibody (IgG1) against PNA binding receptor Isolated from milk fat-body membrane by lectin affinity chromatography. The antibody reacts with the basal lamina of the efferent duct from normal breast as well as with the surface and cytoplasmic structures of breast cancer cells both in primary tumor and in metastases.

### anti-PNAr-III

A monoclonal antibody (IgG) against PNA receptor isolated from the mucus of ovarian cyst by lectin affinity chromatography. The antibody reacts well with various ovarian cancer types both in the primary tumor and metastases.

### anti-WGA

A monoclonal antibody (IgG) which reacts with wheat germ agglutinin antigen (WGA) lectin. Following incubation with WGA, cells possessing WGA binding receptors on their surface can be sensitized by it.

### anti-T3

Antibodies which react with T lymphocytes. They can be used in bone-marrow transplantations to remove T cells.

### anti-H-antigen

A monoclonal antibody which reacts with the surface antigen of the Y chromosome carring bull sperm used for insemination.

### anti-AFP

A monoclonal antibody which reacts with alpha-foetoprotein producing tumors.

### anti-hCG

A monoclonal antibody which reacts with tumors producing human choriogonadotropine.

### Brief Description of the Drawings

Fig. 1 demonstrates the test result with a conjugate obtained according to the invention.

Fig. 2 shows the results of example 1 carried out with anti-OVA and anti-WGA cells.

### Best Mode of Carrying out the Invention

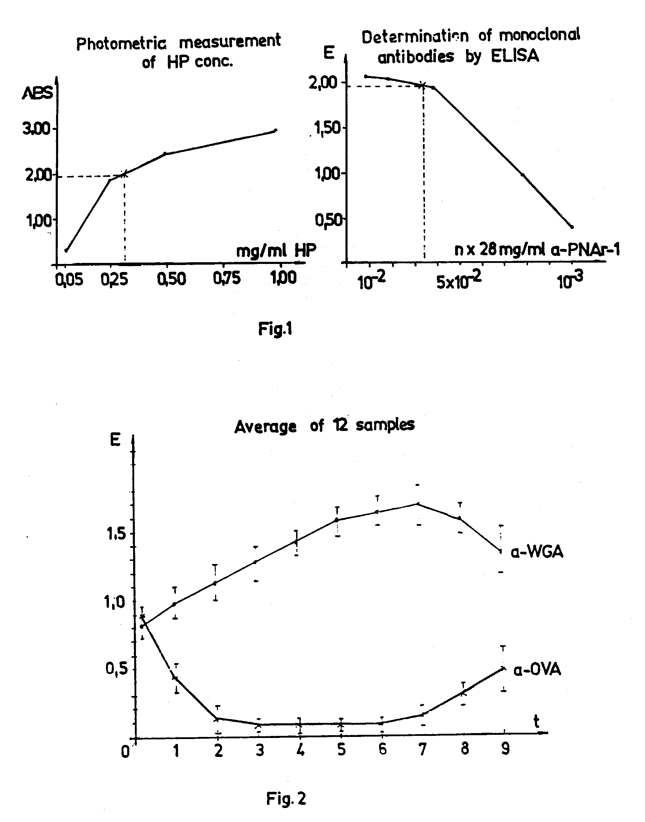

Figure 1 demonstrates the test results with a conjugate produced via the aforementioned method. The anti-PNAr-I monoclonal antibody which was raised against the pea-nut agglutinine antigen-binding receptor present on the surface of normal cells of the gastric mucosa as well as on the neoplastically transformed forms was conjugated with hematoporphyrin followed by freeze-drying. The freeze-dried monoclonal antibody-hematoporphyrin conjugate can be stored indefinitely if desiccated. The conjugate is dissolved in sterile distilled water before use and the specific antibody activity and photosensibiliser binding is determined afterwards.

The conjugate thus prepared is suitable for the purposes of laser photo-immunotargeting.

In an in vitro process a cell mixture is incubated for 1-1.5 hours followed by washing with the culture medium and, afterwards, the mixtures are irradiated by a He-Ne laser at 14 J/cm² irradiation energy at a wavelength practically above 600 nm, preferably at 632 nm wavelength.

Based on our experiments the optimal concentration of the conjugate was 5 microgram /ml hematoporphyrin.

The present invention, therefore, provides in a further aspect the use of the conjugate in a method to remove unwanted cell populations from cell mixtures and tissues.

It occurs several times that separation of certain cell populations (e.g. specific monoclonal antibody-producing hybridomas) is very difficult, moreover, even if the separation is complete the yield of pure cells is very low.

In accordance with the present invention the above problem can be solved by contacting the mixture of the desired and contaminating cells with the conjugate of certain porphyrin derivatives conjugated to the contaminating cell(s)-specific monoclonal antibody and subjecting the mixture to laser irradiation.

By the above invention the ratio of the desired cells can be markedly increased.

The possibilities provided by the invention can be preferably exploited in the production of monoclonal antibodies specific for small molecular weight substances. In these instances the antigen for immunization is usually conjugated to some high molecular weight proteins like thyreoglobuline, bovine serum albumin, key-hole limpit hemocyanine, etc. After cell fusion, therefore, a number of hybridomas occur producing an antibody which reacts with the antigen determinant of the carrier protein. The elimination of such antibodies improves considerably the chances of growing and isolation of the clones.

As already mentioned the conjugate according to the invention is applicable not exclusively in a method for the production of hybridoma cells but, by selecting appropriate monoclonal antibodies, is also suitable in a method for the elimination of T-cells in the course of bone-marrow transplantations and in a method to decrease the concentration of Y-chromosomes in bull sperm used for insemination.

According to the invention, the conjugate is used in an immuno-targeting method wherein living tissue containing non-desired type(s) of cells is treated with the conjugate comprising monoclonal antibodies conjugated with porphyrin derivatives and subsequently irradiated with laser beam of a wavelength higher than that activating the said porphyrin derivative. It is preferably used a He-Ne laser at a wavelength of higher than 600 nm, preferably 630 nm.

In this method the selective disruption of various cells without impairing their environment is of utmost importance. This hypothesis is substantiated by the following experimental findings.

Human gastric cancer cells, as well as mouse hybridoma cells were transplanted dorsally into nude (hairless, thymus deficient) mice by subcutane injection. As soon as the tumor size reached 0.5 cm the conjugate corresponding to 5-10 mg/kg body weight hematoporphyrin was administered. As conjugates anti-PNAr-I monoclonal antibody conjugate for the human tumor bearing mice and WGA-hematoporphyrin complex for mice transplanted with anti-WGA hybridoma were used. After 24 hours the tumors growing on the back of mice were irradiated through the skin by 14 J/cm² energy.

For both models hematoporphyrin conjugate by itself did not influence the growth of the tumor. Likewise, laser irradiation by itself has not changed tumor proliferation. However, on the simultaneous application of both stimuli the tumor was destructed within one day. Following the above treatment, the tumor has completely disappeared leaving behind only callus. The process was monitored also by histological follow-up.

The results lend credit to the applicability of the conjugate also in in vitro phototargeting processes. Their role extends beyond the human health care also to the veterinary field, e.g. to make animals barren.

The present invention is illustrated by the following examples.

### Example 1

A mixture of hybridoma cells is prepared. 1 x 10 anti-WGA cells are mixed at 1:1 ratio with anti-OVA hybridoma cells. The former cell line producing a monoclonal immune gamma globuline specifically reacting with wheat germ agglutinine lectin (WGA) was established in our laboratory in 1984 while the latter cell line was developed by Bötcher et al. in 1978. A HP-anti OVA conjugate corresponding to 5 microgram/ml HP was added to the RPMI-1640 medium and the mixture was incubated for one hour in a CO₂ incubator. The cells were then centrifuged and washed three times in conjugate free RPMI-1640 medium. The cell mixture was irradiated by a He-Ne laser at 14 J/cm² energy. Uniform irradiation could be achieved by a method developed by us earlier by reducing the amount of the culturing medium to the diameter of the laser beam. Before and after irradiation specific antibody production was determined by ELISA and from this the ratio of cell population producing an individual immunoglobuline could be assessed. The results are shown in Figure 2.

As we can see from the Figure 2 the amount of antibody produced by the anti-OVA cells still remained in the cell mixture although their number was very low. The measurable antibody production which was detectable not earlier than one week corresponded to 10 duplications for hybridoma cells which equals to 10-100 surviving cells for 2 x 10⁵ total cell count.

Based on the above results, one single irradiation results in an efficiency orders of magnitude higher compared to the already known cell separation processes like cell sorter.

### Example 2

### Preparation of the conjugate

20.0 mg hematoporphyrin dihydrochloride was dissolved by means of 0.8 ml N,N-dimethyl formamide in 1.25 ml distilled water. This was mixed with 20.0 mg 1-ethyl-3-(3-dimethyl-aminopropyl)carbodiimide hydrochloride (EDCL) dissolved in 0.6 ml of water. After 30 min 15.0 mg antibody protein, dissolved in 5.0 ml distilled water was added to the solution and the mixture was incubated at room temperature for 5 hours while the pH was continuously maintained between 6-7. Thereafter 50 microliter of monoethanol-amine was added and the solution was left at room temperature overnight. Following this step the solution was dialysed against 0.0001 M phosphate buffer for 4 days changing the buffer 3 times daily and finally against PBS overnight (pH was kept always at 7.4). The resulting solution was filtered through Sephadex^{R} G 25. Following the determination of the total protein content the hematoporphyrin binding can be assessed photometrically at 390 nm wavelength by a calibration curve. The activity of the specific antibody can be determinded by immunoserology (e.g. by ELISA).

## Claims (Claims for the following Contracting State(s): BE, CH, DE, FR, GB, IT, LI, LU, NL, SE)

1. A conjugate comprising a monoclonal antibody and a tag characterized in that the tag is hematoporphyrin.

2. A conjugate according to claim 1 in which the monoclonal antibody is selected from anti-PNArI, anti-PNAr-II, anti-PNAr-III, anti-AFP, anti-hCG, anti-WGA, anti-T3 and anti-H.

3. A method for preparing a conjugate according to any of claims 1 or 2 comprising the incubation of a monoclonal antibody with hematoporphyrin in an aqueous medium.

4. A conjugate as claimed in any of claims 1 to 2 or prepared as claimed in claim 3 for use in a method for destroying unwanted cells within a cell population comprising contacting the cell population with a conjugate and subsequently applying a laser irradiation at above 600 nm wavelength.

5. A conjugate as claimed in claim 4 for use in a method for destroying unwanted cells within a cell population comprising contacting the cell population with a conjugate and subsequently applying a laser irradiation at about 630 nm wavelength.

6. A method for destroying in vitro unwanted cells within a cell population comprising contacting the cell population with a conjugate according to claims 1 or 2 or prepared according to claim 3 and subsequently applying a laser irradiation at above 600 nm preferably at 630 nm.

## Claims (Claims for the following Contracting State(s): AT)

1. A method for preparing a conjugate comprising a monoclonal antibody and a tag wherein the tag is hematoporphyrin comprising the incubation of a monoclonal antibody with hematoporphyrin in an aqueous medium.

2. A method as claimed in claim 1 wherein the monoclonal antibody is selected from anti-PNArI, anti-PNAr-II, anti-PNAr-III, anti-AFP, anti-hCG, anti-WGA, anti-T3 and anti-H.

3. A conjugate prepared as claimed in claim 1 or 2 for use in a method for destroying unwanted cells within a cell population comprising contacting the cell population with a conjugate and subsequently applying a laser irradiation at above 600 nm wavelength.

4. A conjugate prepared as claimed in claim 1 or 2 for use in a method for destroying unwanted cells within a cell population comprising contacting the cell population with a conjugate and subsequently applying a laser irradiation at about 630 nm wavelength.

5. A method for destroying in vitro unwanted cells within a cell population comprising contacting the cell population with a conjugate prepared according to claims 1 or 2 and subsequently applying a laser irradiation at above 600 nm preferably at 630 nm.

## Patentansprüche (Patentansprüche für folgende(n) Vertragsstaat(en): BE, CH, DE, FR, GB, IT, LI, LU, NL, SE)

1. Konjugat enthaltend einen monoklonalen Antikörper und eine Markierung, dadurch gekennzeichnet, daß die Markierung Hematoporphyrin ist.

2. Konjugat nach Anspruch 1, in dem der monoklonale Antikörper aus Anti-PNArI, Anti-PNAr-II, Anti-PNAr-III, Anti-AFP, Anti-hCG, Anti-WGA, Anti-T3 und Anti-H ausgewählt ist.

3. Verfahren zur Herstellung eines Konjugats nach einem der Ansprüche 1 oder 2, bei dem man einen monoklonalen Antikörper mit Hematoporphyrin in einem wäßrigen Medium inkubiert.

4. Konjugat nach einem der Ansprüche 1 oder 2 oder hergestellt nach Anspruch 3, zur Verwendung in einem Verfahren zur Zerstörung von unerwünschten Zellen innerhalb einer Zellpopulation, bei dem man die Zellpopulation mit einem Konjugat in Kontakt bringt und anschließend mit Laserlicht bei einer Wellenlänge über 600 nm bestrahlt.

5. Konjugat nach Anspruch 4, zur Verwendung in einem Verfahren zur Zerstörung von unerwünschten Zellen innerhalb einer Zellpopulation, bei dem man die Zellpopulation mit einem Konjugat in Kontakt bringt und anschließend mit Laserlicht bei etwa 630 nm Wellenlänge bestrahlt.

6. Verfahren zur Zerstörung von unerwünschten Zellen innerhalb einer Zellpopulation in vitro, bei dem man die Zellpopulation mit einem Konjugat nach Anspruch 1 oder 2 oder hergestellt nach Anspruch 3 in Kontakt bringt und anschließend mit Laserlicht über 600 nm, vorzugsweise bei 630 nm bestrahlt.

## Patentansprüche (Patentansprüche für folgende(n) Vertragsstaat(en): AT)

1. Verfahren zur Herstellung eines Konjugates enthaltend einen monoklonalen Antikörper und eine Markierung, worin die Markierung Hematoporphyrin ist, bei dem man einen monoklonalen Antikörper mit Hematoporphyrin in einem wäßrigen Medium inkubiert.

2. Verfahren nach Anspruch 1, in dem der monoklonale Antikörper aus Anti-PNAr-I, Anti-PNAr-II, Anti-PNAr-III, Anti-AFP, Anti-hCG, Anti-WGA, Anti-T3 und Anti-H ausgewählt ist.

3. Konjugat hergestellt nach einem der Ansprüche 1 oder 2 zur Verwendung in einem Verfahren zur Zerstörung von unerwünschten Zellen innerhalb einer Zellpopulation, bei dem man die Zellpopulation mit einem Konjugat in Kontakt bringt und anschließend mit Laserlicht bei einer Wellenlänge über 600 nm bestrahlt.

4. Konjugat hergestellt nach Anspruch 1 oder 2 zur Verwendung in einem Verfahren zur Zerstörung von unerwünschten Zellen innerhalb einer Zellpopulation, bei dem man die Zellpopulation mit einem Konjugat in Kontakt bringt und anschließend mit Laserlicht bei etwa 630 nm Wellenlänge bestrahlt.

5. Verfahren zur Zerstörung von unerwünschten Zellen innerhalb einer Zellpopulation in vitro, bei dem man die Zellpopulation mit einem Konjugat hergestellt nach Anspruch 1 oder 2 in Kontakt bringt und anschließend mit Laserlicht über 600 nm, vorzugsweise bei 630 nm, bestrahlt.

## Revendications (Revendications pour l'(les) Etat(s) contractant(s) suivant(s): BE, CH, DE, FR, GB, IT, LI, LU, NL, SE)

1. Produit conjugué comprenant un anticorps monoclonal et un marqueur caractérisé en ce que ce dernier est l'hématoporphyrine.

2. Produit conjugué selon la revendication 1 dans lequel l'anticorps monoclonal est choisi parmi l'anti-PNArI, l'anti-PNAr-II, l'anti-PNAr-III, l'anti-AFP, l'anti-hCG, l'anti-WGA, l'anti-T3 et l'anti-H.

3. Méthode de préparation d'un produit conjugué selon l'une des revendications 1 ou 2 comprenant l'incubation d'un anticorps monoclonal avec l'hématoporphyrine dans un milieu aqueux.

4. Produit conjugué selon l'une des revendications 1 ou 2 ou préparé selon la revendication 3 à utiliser dans une méthode de destruction de cellules indésirées dans une population cellulaire comprenant la mise en contact de la population cellulaire avec un produit conjugué et l'application subséquente d'un rayonnement laser à une longueur d'ondes supérieure à 600 nm.

5. Produit conjugué selon la revendication 4 à utiliser dans une méthode de destruction de cellules indésirées dans une population cellulaire comprenant la mise en contact de la population cellulaire avec un produit conjugué et l'application subséquente d'un rayonnement Laser à une longueur d'ondes d'environ 630 nm.

6. Méthode de destruction in vitro de cellules indésirées dans une population cellulaire comprenant la mise en contact de la population cellulaire avec un produit conjugué selon les revendications 1 ou 2 ou préparé selon la revendication 3 et l'application subséquente d'un rayonnement laser à une longueur d'ondes supérieure à 600 nm, de préférence de 630 nm.

## Revendications (Revendications pour l'(les) Etat(s) contractant(s) suivant(s): AT)

1. Méthode de préparation d'un produit conjugué comprenant un anticorps monoclonal et un marqueur, ce dernier étant L'hématoporphyrine comprenant L'incubation d'un anticorps monoclonal avec l'hématoporphyrine dans un milieu aqueux.

2. Méthode selon la revendication 1 dans laquelle l'anticorps monoclonal est choisi parmi l'anti-PNArI, l'anti-PNAr-II, l'anti-PNAr-III, l'anti-AFP, l'anti-hCG, l'anti-WGA, l'anti-T3 et l'anti-H.

3. Produit conjugué préparé selon l'une des revendications 1 ou 2 à utiliser dans une méthode de destruction de cellules indésirées dans une population cellulaire comprenant la mise en contact de la population cellulaire avec un produit conjugué et l'application subséquente d'un rayonnement laser à une longueur d'ondes supérieure à 600 nm.

4. Produit conjugué préparé selon les revendications 1 ou 2 à utiliser dans une méthode de destruction de cellules indésirées dans une population cellulaire comprenant la mise en contact de la population cellulaire avec un produit conjugué et l'application subséquente d'un rayonnement laser à une longueur d'ondes d'environ 630 nm.

5. Méthode de destruction in vitro de cellules indésirées dans une population cellulaire comprenant la mise en contact de la population cellulaire avec un produit conjugué préparé selon les revendications 1 ou 2 et l'application subséquente d'un rayonnement laser à une longueur d'ondes supérieure à 600 nm, de préférence de 630 nm.
